# EUROPÄISCHE PATENTSCHRIFT

(11) **EP 1 266 668 B1**
(45) Veröffentlichungstag und Bekanntmachung des Hinweises auf die Patenterteilung: **13.10.2004**
(21) Anmeldenummer: 02011718.0
(22) Anmeldetag: 25.05.2002
(51) Int. Cl.: A61M 1/16

(54) **Verfahren zum Nachweis von Verunreinigungen in einer Spülflüssigkeit zum Spülen einer extrakorporalen Blutbehandlungsvorrichtung und Vorrichtung**
Method for detection of impurities in a rinsing liquid for rinsing an extracorporeal blood treatment system and apparatus
Méthode de détection de la présence de contamination dans le liquide de rinçage pour un système de traitement extracorporel du sang, et appareil

(30) Priorität: 12.06.2001 DE 10128278
(43) Veröffentlichungstag der Anmeldung: 18.12.2002
(73) Patentinhaber: Fresenius Medical Care Deutschland GmbH, 61352 Bad Homburg v.d.H. (DE)
(72) Erfinder: Balschat, Klaus, 97525 Schwebheim (DE); Spickermann, Reiner, 97535 Wasserlosen-Burghausen (DE)
(74) Vertreter: Luderschmidt, Schüler & Partner GbR

(56) Entgegenhaltungen:
- DE-A- 3 442 744
- US-A- 4 695 385

## Beschreibung

Die vorliegende Erfindung betrifft eine Vorrichtung zur extrakorporalen Blutbehandlung mit einer Einrichtung zum Nachweis von Verunreinigungen in einer Spülflüssigkeit zum Spülen des Flüssigkeitssystems der extrakorporalen Blutbehandlungsvorrichtung. Darüber hinaus bezieht sich die Erfindung auf ein Verfahren zum Nachweis von Verunreinigungen in einer Spülflüssigkeit zum Spülen einer extrakorporalen Blutbehandlungsvonichtung.

Die bekannten Blutbehandlungsvorrichtungen verfügen über eine Einrichtung zum Spülen und Desinfizieren des Flüssigkeitssystems. Es ist bekannt, die Blutbehandlungsvorrichtungen zum Spülen in einen Spülmodus zu schalten, in dem Spülflüssigkeit in das Flüssigkeitssystem strömt. Zum Desinfizieren werden die Blutbehandlungsvorrichtungen in einen Rezirkulationsmodus geschaltet, in dem Desinfektionsmittel in dem Flüssigkeitssystem rezirkuliert. Spülflüssigkeit und Desinfektionsmittel werden den bekannten Blutbehandlungsvorrichtungen im allgemeinen über externe Anschlüsse zugeführt.

Für die Sicherheit der Blutbehandlungsvorrichtungen ist von entscheidender Bedeutung, daß nach dem Spülen keine Desinfektionsmittelrückstände in dem Flüssigkeitssystem verbleiben. Die Überprüfung des Spülvorgangs erfolgt bei einigen Maschinen mittels Teststreifen oder -stäbchen, die einen Nachweis von Desinfektionsmittelrückständen erlauben. Dieser manuelle Test erweist sich aber als relativ aufwendig.

Die US-A-4,695,385 beschreibt ein System zum Spülen und Desinfizieren einer Dialysevorrichtung, bei dem die Leitfähigkeit der aus dem Dialysator strömenden Flüssigkeit überwacht wird, um festzustellen, ob sich Spülflüssigkeit oder Desinfektionsmittel im Flüssigkeitssystem der Dialysevorrichtung befindet.

Verfahren zur Überwachung der Zusammensetzung der Dialysierflüssigkeit, die auf einer Leitfähigkeitsmessung beruhen, sind beispielsweise aus der EP-A-0 311 848 und EP-A-0 443 324 bekannt.

Die DE 34 42 744 A beschreibt eine Vorrichtung und ein Verfahren zum Nachweis von Reinigungs- oder Desinfektionsmittelkonzentrat im Dialysierflüssigkeitsweg einer extrakorporalen Blutbehandlungsvorrichtung. Zum Nachweis der Verunreinigungen kommt ein leitfähiges Reinigungs- oder Desinfektionsmittelkonzentrat zum Einsatz. Der Dialysierflüssigkeitsweg wird solange mit Wasser freigespült, bis sich die mit einem Leitfähigkeitsmesser im Dialysierflüssigkeitsweg gemessene Leitfähigkeit des Wassers auf einen Basiswert nahe Null einstellt. Zu diesem Zeitpunkt wird angenommen, dass der Dialysierflüssigkeitsweg nicht mehr mit Reinigungs- oder Desinfektionsmittelkonzentrat verunreinigt ist. Beim Freispülen strömt frisches Wasser als Spülflüssigkeit in den Dialysierflüssigkeitsweg. Die verbrauchte Spülflüssigkeit wird verworfen. Da der Referenzwert, mit dem die Dialysierflüssigkeit gemessene Leitfähigkeit verglichen wird, ein Wert ist, der fest vorgegeben ist, ist es nicht möglich, in einer anderen Spülflüssigkeit, deren Leitfähigkeit nicht bekannt ist, Verunreinigungen nachzuweisen.

Der Erfindung liegt die Aufgabe zugrunde, eine Vorrichtung zur extrakorporalen Blutbehandlung bereitzustellen, die mit großer Sicherheit und relativ geringem konstruktiven Aufwand die Feststellung eines Störfalls aufgrund von Verunreinigungen im Flüssigkeitssystem erlaubt.

Eine weitere Aufgabe der Erfindung ist, ein Verfahren anzugeben, dass die Erkennung eines Störfalls aufgrund von Verunreinigungen im Flüssigkeitssystem einer extrakorporalen Blutbehandlungsvorrichtung mit großer Sicherheit und verhältnismäßig geringem Aufwand ermöglicht.

Die Lösung dieser Aufgaben erfolgt erfindungsgemäß mit den Merkmalen des Patentanspruchs 1 bzw. 8.

Die Erkennung eines Störfalls aufgrund von Verunreinigungen im Flüssigkeitssystem beruht bei der erfindungsgemäßen Blutbehandlungsvorrichtung bzw. dem erfindungsgemäßen Verfahren auf einer Vergleichsmessung einer physikalischen und/oder chemischen Eigenschaft der Spülflüssigkeit. Als physikalische und/oder chemische Eigenschaft kommen alle Messgrößen in Frage, die dem Nachweis von Rückständen in der Spülflüssigkeit dienen.

Die physikalische und/oder chemische Eigenschaft der Spülflüssigkeit wird zunächst in einem Spülmodus gemessen, in dem Spülflüssigkeit in das Flüssigkeitssystem strömt. Dabei wird davon ausgegangen, dass frische Spülflüssigkeit zufließt, d. h. die Spülflüssigkeit nicht verunreinigt ist. Die Messung im Spülmodus liefert einen Referenzwert für die Messung der physikalischen und/oder chemischen Eigenschaft der Spülflüssigkeit in einem nachfolgenden Rezirkulationsmodus, in dem Spülflüssigkeit in dem Flüssigkeitssystem rezirkuliert. Die Messung im Rezirkulationsmodus kann nach jeder Spülung oder Desinfektion des Dialysierflüssigkeitssystems erfolgen, um zu erkennen, ob das Flüssigkeitssystem restlos von den Verunreinigungen befreit ist.

Die Messung im Rezirkulationsmodus liefert einen Vergleichswert, der mit dem Referenzwert verglichen wird. Wenn der Vergleichswert gleich dem Referenzwert ist, kann davon ausgegangen werden, daß das Flüssigkeitssystem ausreichend gespült ist. Dabei ist es möglich, einen Grenzwert zu definieren, der einen gewissen Grad von Verunreinigungen zuläßt.

Von Vorteil ist, daß keine absoluten, sondern nur relative Werte gemessen werden, wodurch die Genauigkeit der Messung beträchtlich erhöht wird. Darüber hinaus wird durch die Rezirkulation eine Totraumbildung vermieden, und durch die Aufkonzentration etwaiger Rückstände während der Rezirkulation können auch kleinste Verunreinigungen sicher erkannt werden. Vorteilhaft ist auch, daß für beide Messungen nur eine Meßeinheit erforderlich ist.

Unter Blutbehandlungsvorrichtung wird jede Vorrichtung verstanden, die eine Behandlung des Bluts eines Patienten in einem extrakorporalen Kreislauf erlaubt. Insbesondere zählen zu den Blutbehandlungsvorrichtungen die bekannten Hämodialyse-, Hämofiltrations- und Hämodiafiltrationsvorrichtungen.

Die Messung der physikalischen und/oder chemischen Eigenschaft der Spülflüssigkeit erfolgt vorzugsweise im Spülmodus in der zufließenden frischen Spülflüssigkeit, die in das Flüssigkeitssystem strömt. Prinzipiell ist es aber auch möglich, die physikalische und/oder chemische Eigenschaft der durch das Flüssigkeitssystem oder aus dem Flüssigkeitssystem strömenden Spülflüssigkeit zu messen. Dies setzt aber voraus, daß das Flüssigkeitssystem vor der Messung vollständig von Verunreinigungen befreit worden ist.

In einer bevorzugten Ausgestaltung wird nach Beendigung der Blutbehandlung das Flüssigkeitssystem im Spülmodus mit Spülflüssigkeit freigespült, wobei die physikalische und/oder chemische Eigenschaft der Spülflüssigkeit als Referenzwert gemessen wird. Anschließend wird das Flüssigkeitssystem in einen ersten Rezirkulationsmodus umgeschaltet, in dem Desinfektionsmittel in dem Flüssigkeitssystem rezirkuliert. In einem anschließenden Spülmodus wird das Flüssigkeitssystem dann mit Spülflüssigkeit zwangsgespült. Nun wird das Flüssigkeitssystem zum Rezirkulieren von Spülflüssigkeit in einen zweiten Rezirkulationsmodus umgeschaltet, um die physikalische und/oder chemische Eigenschaft der Spülflüssigkeit messen und mit dem zuvor gemessenen Referenzwert vergleichen zu können.

Die zu messende physikalische und/oder chemische Eigenschaft ist vorzugsweise die Leitfähigkeit der Spülflüssigkeit. Die Messung dieser Eigenschaft kann mit den bekannten Leitfähigkeitssensoren erfolgen, die ohnehin in den Blutbehandlungsvorrichtungen vorgesehen sind. Anstelle von Leitfähigkeitssensoren können aber auch Ultraschallsensoren oder optische Sensoren zum Nachweis von Verunreinigungen Verwendung finden.

Als Spülflüssigkeit wird vorzugsweise Wasser verwendet, das der Blutbehandlungsvorrichtung zugeführt wird.

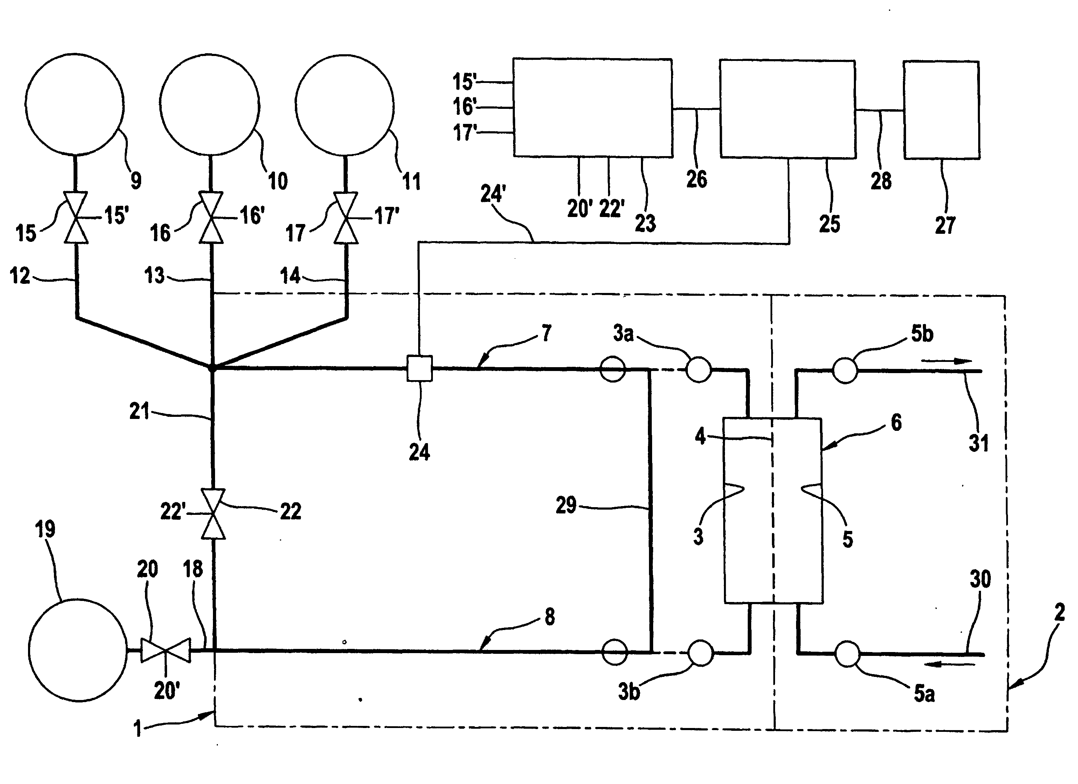

Im folgenden wird ein Ausführungsbeispiel der Erfindung anhand der Zeichnung näher erläutert, die in stark vereinfachter Darstellung die wesentlichen Baugruppen einer Hämodialysevorrichtung zeigt.

Die Hämodialysevorrichtung verfügt über ein Dialysierflüssigkeitssystem 1 und einen extrakorporalen Blutkreislauf 2. Das Dialysierflüssigkeitssystem umfaßt die Dialysierflüssigkeitskammer 3 eines durch eine semipermeable Membran 4 in die Dialysierflüssigkeitskammer und eine Blutkammer 5 unterteilten Dialysators 6 sowie einen ersten Flüssigkeitsweg 7 zum Zuführen von Flüssigkeit zu der Dialysierflüssigkeitskammer 3 und einen zweiten Flüssigkeitsweg 8 zum Abführen von Flüssigkeit aus der Dialysierflüssigkeitskammer des Dialysators 6.

An den ersten Flüssigkeitsweg 7 sind eine Dialysierflüssigkeitsquelle 9 zur Bereitstellung von frischer Dialysierflüssigkeit, eine Spülflüssigkeitsquelle 10 zur Bereitstellung einer Spülflüssigkeit, insbesondere Wasser, und eine Desinfektionsmittelquelle 11 zur Bereitstellung eines Desinfektionsmittels, beispielsweise Natriumhypochlorit oder Formaldehyd über Schlauchleitungen 12, 13, 14 angeschlossen, in denen jeweils ein elektromagnetisch oder pneumatisch betätigbares Ventil 15, 16, 17 zum Öffnen bzw. Schließen der Leitungen angeordnet sind. Der zweite Flüssigkeitsweg 8 führt über eine Leitung 18 zu einem Abfluß 19, in der ebenfalls ein elektromagnetisch oder pneumatisch betätigbares Ventil 20 angeordnet ist. Zum Kurzschließen der beiden Flüssigkeitswege 7, 8 ist eine Bypassleitung 21 vorgesehen, in der wiederum ein elektromagnetisch oder pneumatisch betätigbares Ventil 22 angeordnet ist. Alle Ventile werden über entsprechende Steuerleitungen 15' bis 22' von einer Steuereinheit 23 angesteuert.

In dem ersten Flüssigkeitsweg 7 ist stromab der Bypassleitung 21 ein Leitfähigkeitssensor 24 angeordnet, der über eine Datenleitung 24' mit einer Auswerteinheit 25 verbunden ist. Die Auswerteinheit ist über eine Datenleitung 26 wiederum mit der Steuereinheit 23 verbunden. Eine Alarmeinheit 27, die einen akustischen und/oder optischen Alarm gibt, ist über eine Signalleitung 28 an der Auswerteinheit angeschlossen.

Im Spül- und Rezirkulationsmodus ist der Dialysator 6 abgetrennt und die beiden Flüssigkeitswege 7, 8 sind mit einem Kurzschlußstück 29 verbunden. Im Dialysierbetrieb hingegen ist der erste Flüssigkeitsweg 7 an dem Einlaß 3a und der zweite Dialysierflüssigkeitsweg 8 an dem Auslaß 3b der Dialysierflüssigkeitskammer 3 des Dialysators 6 angeschlossen.

Der extrakorporale Blutkreislauf weist eine zu dem Einlaß 5a der Blutkammer 5 führende Blutzuführleitung 30 und eine von dem Auslaß 5b der Blutkammer 5 abgehende Blutrückführleitung 31 auf.

Darüber hinaus verfügt die Hämodialysevorrichtung noch über weitere Einreichungen, beispielsweise Pumpen zum Fördern der Flüssigkeiten, eine Bilanziereinheit zum Bilanzieren frischer gegenüber verbrauchter Dialysierflüssigkeit, eine Entgasungs- und Heizeinheit, Tropfkammer etc., die aber der besseren Übersichtlichkeit halber nicht dargestellt sind.

Die Funktionsweise der Dialysevorrichtung wird nachfolgend erläutert. Im Dialysierbetrieb strömt Dialysierflüssigkeit aus der Dialysierflüssigkeitsquelle 9 über den ersten Dialysierflüssigkeitsweg 7 in die Dialysierflüssigkeitskammer 3 des Dialysators 6 und aus der Dialysierflüssigkeitskammer über den zweiten Flüssigkeitsweg 8 in den Abfluß 20. Hierzu öffnet die Steuereinheit 23 die Ventile 15, 20 und schließt die Ventile 16, 17, 22.

Nach der Dialysebehandlung erfolgt eine Freispülung des Dialysierflüssigkeitssystems 1. Hierzu schaltet die Steuereinheit das Flüssigkeitssystem 1 in den Spülmodus, in dem die Ventile 15, 17, 22 geschlossen und die Ventile 16, 20 geöffnet sind, wobei der Dialysator 6 durch das Kurzschlußstück 29 ersetzt wird. Der Leitfähigkeitsmesser 24 mißt die Leitfähigkeit der frischen Spülflüssigkeit, die aus der Spülflüssigkeitsquelle 10 in den ersten Flüssigkeitsweg 7 strömt und über den zweiten Flüssigkeitsweg 8 in den Abfluß 19 abfließt. Die gemessene Leitfähigkeit wird in der Auswerteinheit 25 als Referenzwert gespeichert.

Zur Desinfektion wird aus der Desinfektionsmittelquelle 11 Desinfektionsmittel in das Dialysierflüssigkeitssystem 1 geleitet. Nachdem das Flüssigkeitssystem 1 mit Desinfektionsmittel befüllt ist, schaltet die Steuereinheit 23 das Flüssigkeitssystem in einen ersten Rezirkulationsmodus um, in dem die Ventile 15, 16, 17, 20 geschlossen und das Ventil 22 geöffnet sind. Das Desinfektionsmittel rezirkuliert nun über den ersten und zweiten Leitungszweig 7, 8 und die Bypassleitung 21.

Anschließend wird das Dialysierflüssigkeitssystem 1 zwangsgespült. Die Steuereinheit 23 schaltet das Flüssigkeitssystem wieder in den Spülmodus um, in dem die Ventile 15, 17, 22 geschlossen und die Ventile 16, 20 geöffnet sind. Die Spülflüssigkeit strömt nun wieder durch den ersten und zweiten Leitungszweig 7, 8 in den Ablauf 19. Die Zwangsspülung erfolgt solange, bis das Flüssigkeitssystem vollständig von allen Desinfektionsmittelrückständen befreit ist. Dies wird nun in dem folgenden zweiten Rezirkulationsmodus überprüft.

Nach Umschalten in den zweiten Rezirkulationsmodus, in dem die Ventile 15, 16, 17, 20 geschlossen und das Ventil 22 geöffnet sind, wird die Leitfähigkeit der in dem Dialysierflüssigkeitssystem rezirkulierenden Spülflüssigkeit mit dem Leitfähigkeitssensor 24 gemessen. Der Meßwert wird in der Auswerteinheit 25 mit dem zuvor gewonnenen Referenzwert verglichen. Wenn der Betrag der Differenz von Meßwert und Referenzwert größer als ein vorgegebener Grenzwert ist, erzeugt die Auswerteinheit ein Steuersignal, daß die Alarmeinheit 27 über die Datenleitung 28 empfängt. Der Grenzwert ist vorzugsweise "0". Wenn geringfügige Desinfektionsmittelrückstände zulässig sein sollen, kann der Betrag des Grenzwerts auch größer "0" sein; er sollte jedenfalls sehr klein sein. Die Alarmeinheit 27 gibt dann einen akustischen und/oder optischen Alarm, um den Störfall anzuzeigen. Das Steuersignal der Auswerteinheit kann aber auch zur Einleitung eines Eingriffs in die Steuerung der Dialysevorrichtung herangezogen werden. Beispielsweise kann ein neuer Spülvorgang eingeleitet oder die Maschine abgeschaltet werden. Anstelle einer Messung können auch mehrere Messungen im Rezirkulationsmodus erfolgen, wobei die einzelnen Meßwerte dann mit dem Referenzwert verglichen werden. Auch der Referenzwert kann durch Mittelwertbildung aus mehreren Messungen im Spülmodus gewonnen werden.

## Patentansprüche

1. Vorrichtung zur extrakorporalen Blutbehandlung mit
einem Flüssigkeitssystem (1),
einer Steuereinheit (23) zum Umschalten des Flüssigkeitssystems in einen Spülmodus, in dem Flüssigkeit in das Flüssigkeitssystem strömt und einen Rezirkulationsmodus, in dem Flüssigkeit in dem Flüssigkeitssystem rezirkuliert,
einer Messeinheit (24) zum Messen einer physikalischen und/oder chemischen Eigenschaft einer Spülflüssigkeit, wobei die physikalische und/oder chemische Eigenschaft der Spülflüssigkeit dem Nachweis von Verunreinigungen in der Flüssigkeit dient,
einer Auswerteinheit (25) zum Vergleichen der von der Messeinheit (24) gemessenen physikalischen und/oder chemischen Eigenschaft der Spülflüssigkeit mit einem Referenzwert, wobei die Auswerteinheit auf einen Störfall schließt, wenn der Betrag der Differenz größer als ein vorgegebener Grenzwert ist, **dadurch gekennzeichnet,**
**dass** der Referenzwert die von der Messeinheit (24) im Spülmodus gemessene physikalische und/oder chemische Eigenschaft der Spülflüssigkeit ist, wobei die Auswerteinheit den Referenzwert mit der von der Messeinheit im Rezirkulationsmodus gemessenen physikalischen und/oder chemischen Eigenschaft der Spülflüssigkeit vergleicht.

2. Vorrichtung nach Anspruch 1, **dadurch gekennzeichnet, dass** das Flüssigkeitssystem (1) einen Flüssigkeitsweg (7) zum Zuführen von Flüssigkeit in das Flüssigkeitssystem und einen Flüssigkeitsweg (8) zum Abführen von Flüssigkeit aus dem Flüssigkeitssystem aufweist, wobei die Steuereinheit (23) zum Umschalten in den Rezirkulationsmodus den Flüssigkeitsweg zum Zuführen von Flüssigkeit mit dem Flüssigkeitsweg zum Abführen von Flüssigkeit verbindet.

3. Vorrichtung nach Anspruch 2, **dadurch gekennzeichnet, dass** die Messeinheit (24) in dem Flüssigkeitsweg (7) zum Zuführen von Flüssigkeit angeordnet ist.

4. Vorrichtung nach einem der Ansprüche 1 bis 3, **dadurch gekennzeichnet, dass** Mittel (10, 13, 16) zum Zuführen von Spülflüssigkeit und Mittel (11, 14, 17) zum Zufuhren von Desinfektionsmittel in das Flüssigkeitssystem (1) vorgesehen sind, wobei die Mittel zum Zuführen der Spülflüssigkeit und die Mittel zum Zuführen von Desinfektionsmittel von der Steuereinheit (23) derart ansteuerbar sind, dass das Flüssigkeitssystem zum Freispülen mit Spülflüssigkeit in den Spülmodus, zum Rezirkulieren von Desinfektionsmittel in einen ersten Rezirkulationsmodus, zum Zwangsspülen mit Spülflüssigkeit in den Spülmodus und zum Rezirkulieren von Spülflüssigkeit in einen zweiten Rezirkulationsmodus geschaltet wird.

5. Vorrichtung nach einem der Ansprüche 1 bis 4, **dadurch gekennzeichnet, dass** die in dem Flüssigkeitssystem nachzuweisende Verunreinigung Desinfektionsmittel ist.

6. Vorrichtung nach einen der Ansprüche 1 bis 5, **dadurch gekennzeichnet, dass** die Messeinheit (24) ein Leitfähigkeitsmesser ist.

7. Vorrichtung nach einem der Ansprüche 1 bis 6, **dadurch gekennzeichnet, dass** die Spülflüssigkeit Wasser ist.

8. Verfahren zum Nachweis von Verunreinigungen in einer Spülflüssigkeit zum Spülen eines Flüssigkeitssystems einer Vorrichtung zur extrakorporalen Blutbehandlung, das in einen Spülmodus, in dem Flüssigkeit in das Flüssigkeitssystem strömt und einen Rezirkulationsmodus umschaltbar ist, in dem die Flüssigkeit in dem Flüssigkeitssystem rezirkuliert, wobei zum Nachweis der Verunreinigungen eine physikalische und/oder chemische Eigenschaft der Spülflüssigkeit gemessen, mit einem Referenzwert verglichen und auf einen Störfall geschlossen wird, wenn der Betrag der Differenz größer als ein vorgegebener Grenzwert ist, **dadurch gekennzeichnet, dass** der Referenzwert die im Spülmodus gemessene physikalische und/oder chemische Eigenschaft der Spülflüssigkeit ist, wobei der Referenzwert mit der im Rezirkulationsmodus gemessenen physikalischen und/oder chemischen Eigenschaft der Spülflüssigkeit verglichen wird.

9. Verfahren nach Anspruch 8, **dadurch gekennzeichnet, dass** im Spülmodus die physikalische und/oder chemische Eigenschaft der dem Flüssigkeitssystem zugeführten Spülflüssigkeit gemessen wird.

10. Verfahren nach Anspruch 8 oder 9, **dadurch gekennzeichnet, dass** das Flüssigkeitssystem zum Freispülen mit Spülflüssigkeit in den Spülmodus geschaltet und die physikalische und/oder chemische Eigenschaft der Spülflüssigkeit gemessen wird, zum Rezirkulieren von Desinfektionsmittel in einen ersten Rezirkulationsmodus und zum Zwangsspülen mit Spülflüssigkeit in den Spülmodus geschaltet wird, und zum Rezirkulieren von Spülflüssigkeit in einen zweiten Rezirkulationsmodus geschaltet und die physikalische und/oder chemische Eigenschaft der Spülflüssigkeit gemessen wird.

11. Verfahren nach einem der Ansprüche 8 bis 10, **dadurch gekennzeichnet, dass** die in dem Flüssigkeitssystem nachzuweisende Verunreinigung Desinfektionsmittel ist.

12. Verfahren nach einem der Ansprüche 8 bis 11, **dadurch gekennzeichnet, dass** die physikalische und/oder chemische Eigenschaft die Leitfähigkeit der Spülflüssigkeit ist.

13. Verfahren nach einem der Ansprüche 8 bis 12, **dadurch gekennzeichnet, dass** die Spülflüssigkeit Wasser ist.

## Claims

1. A device for extracorporeal blood treatment with
a liquid system (1),
a control unit (23) for switching the liquid system into a rinsing mode, in which liquid flows into the liquid system, and a recirculation mode, in which liquid recirculates in the liquid system,
a measuring unit (24) for measuring a physical and/or chemical property of a rinsing liquid, whereby the physical and/or chemical property of the rinsing liquid is used to detect impurities in the liquid,
an evaluation unit (25) for comparing the physical and/or chemical property of the rinsing liquid measured by the measuring unit (24) with a reference value, whereby the evaluation unit concludes that there is a malfunction when the amount of the difference is greater than a preset limiting value, **characterised in that**
the reference value is the physical and/or chemical property of the rinsing liquid measured by the measuring unit (24) in the rinsing mode, whereby the evaluation unit compares the reference value with the physical and/or chemical property of the rinsing liquid measured by the measuring unit in the recirculation mode.

2. The device according to claim 1, **characterised in that** the liquid system (1) has a liquid path (7) for the feeding of liquid into the liquid system and a liquid path (8) for the removal of liquid from the liquid system, whereby the control unit (23) for switching into the recirculation mode connects the liquid path for the feeding of liquid with the liquid path for the removal of liquid.

3. The device according to claim 2, **characterised in that** the measuring unit (24) is arranged in the liquid path (7) for the feeding of liquid.

4. The device according to any one of claims 1 to 3, **characterised in that** means (10, 13, 16) for the feeding of rinsing liquid and means (11, 14, 17) for the feeding of disinfectant into the liquid system (1) are provided, whereby the means for the feeding of rinsing liquid and the means for the feeding of disinfectant can be controlled by the control unit (23) in such a way that the liquid system is switched into the rinsing mode for the free-rinsing with rinsing liquid, into a first recirculation mode for the recirculation of disinfectant, into the rinsing mode for the forced-rinsing with rinsing liquid and into a second recirculation mode for the recirculation of rinsing liquid.

5. The device according to any one of claims 1 to 4, **characterised in that** the impurity to be detected in the liquid system is disinfectant

6. The device according to any one of claims 1 to 5, **characterised in that** the measuring unit (24) is a conductivity meter.

7. The device according to any one of claims 1 to 6, **characterised in that** the rinsing liquid is water.

8. A method for the detection of impurities in a rinsing liquid for the rinsing of a liquid system of a device for extracorporeal blood treatment, which can be switched into a rinsing mode, in which liquid flows into the liquid system, and a recirculation mode, in which the liquid recirculates in the liquid system, whereby, for the detection of impurities, a physical and/or chemical property of the rinsing liquid is measured and compared with a reference value and it is concluded that there is a malfunction if the amount of the difference is greater than a preset limiting value, **characterised in that** the reference value is the physical and/or chemical property of the rinsing liquid measured in the rinsing mode, whereby the reference value is compared with the physical and/or chemical property of the rinsing liquid measured in the recirculation mode.

9. The method according to claim 8, **characterised in that** the physical and/or chemical property of the rinsing liquid fed to the rinsing system is measured in the rinsing mode.

10. The method according to claim 8 or 9, **characterised in that** the liquid system is switched into the rinsing mode for the free-rinsing with rinsing liquid and the physical and/or chemical property of the rinsing liquid is measured, is switched into a first recirculation mode for the recirculation of disinfectant and into the rinsing mode for the forced-rinsing with rinsing liquid, and is switched into a second recirculation mode for the recirculation of rinsing liquid and the physical and/or chemical property of the rinsing liquid is measured.

11. The method according to any one of claims 8 to 10, **characterised in that** the impurity to be detected in the liquid system is disinfectant.

12. The method according to any one of claims 8 to 11, **characterised in that** the physical and/or chemical property is the conductivity of the rinsing liquid.

13. The method according to any one of claims 8 to 12, **characterised in that** the rinsing liquid is water.

## Revendications

1. Dispositif pour le traitement extracorporel du sang avec
un système de liquide (1),
une unité de commande (23) pour commuter le système de liquide dans un mode de rinçage dans lequel du liquide s'écoule dans le système de liquide et dans un mode de recirculation dans lequel du liquide recircule dans du système de liquide,
une unité de mesure (24) pour mesurer une propriété physique et/ou chimique d'un liquide de rinçage, où la propriété physique et/ou chimique du liquide de rinçage sert à la détection d'impuretés dans le liquide,
une unité d'évaluation (25) pour comparer la propriété physique et/ou chimique du liquide de rinçage mesurée par unité de mesure (24) avec une valeur de référence, où l'unité d'évaluation conclut à un événement anormal quand la valeur de la différence est supérieure à une valeur limite prédéterminée, **caractérisé en ce que**
la valeur de référence est la propriété physique et/ou chimique du liquide de rinçage mesurée en mode de rinçage par l'unité de mesure (24), où l'unité d'évaluation compare la valeur de référence avec la propriété physique et/ou chimique du liquide de rinçage mesurée en mode de recirculation par l'unité de mesure.

2. Dispositif selon la revendication 1 **caractérisé en ce que** le système de liquide (1) comporte une voie de liquide (7) pour l'amenée de liquide dans le système de liquide et une voie de liquide (8) pour l'évacuation de liquide du système de liquide, où l'unité de commande (23) relie la voie de liquide pour l'amenée de liquide avec la voie de liquide pour l'évaluation du liquide pour la commutation dans le mode de recirculation.

3. Dispositif selon la revendication 2 **caractérisé en ce que** l'unité de mesure (24) est disposée dans la voie de liquide (7) pour l'amenée de liquide.

4. Dispositif selon l'une des revendications 1 à 3 **caractérisé en ce qu'**il est prévu des moyens (10, 13, 16) pour l'amenée de liquide de rinçage et des moyens (11, 14, 17) pour l'amenée de désinfectant dans le système de liquide (1), où les moyens pour l'amenée du liquide de rinçage et les moyens pour l'amenée du désinfectant peuvent être commandés par l'unité de commande (23) de telle manière que le système de liquide est commuté dans le mode de rinçage pour le rinçage libre avec du liquide de rinçage, dans un premier mode de recirculation pour la recirculation du désinfectant, dans le mode de rinçage pour le rinçage forcé avec du liquide de rinçage et dans un deuxième mode de recirculation pour la recirculation du liquide de rinçage.

5. Dispositif selon l'une des revendications 1 à 4 **caractérisé en ce que** l'impureté à détecter dans le système de liquide est le désinfectant.

6. Dispositif selon l'une des revendications 1 à 5 **caractérisé en ce que** l'unité de mesure (24) est un dispositif de mesure de conductibilité.

7. Dispositif selon l'une des revendications 1 à 6 **caractérisé en ce que** le liquide de rinçage est l'eau.

8. Procédé pour détecter des impuretés dans un liquide de rinçage pour rincer un système de liquide d'un dispositif pour le traitement extracorporel du sang qui peut être commuté dans un mode de rinçage dans lequel un liquide s'écoule dans le système de liquide et dans un mode de recirculation dans lequel le liquide recircule dans le système de liquide, où, pour la détection des impuretés, une propriété physique et/ou chimique du liquide de rinçage est mesurée, comparée avec une valeur de référence et un événement anormal est inféré quand la valeur de la différence est supérieure à une valeur limite prédéterminée, **caractérisé en ce que** la valeur de référence est la propriété physique et/ou chimique du liquide de rinçage mesurée en mode de rinçage, où la valeur de référence est comparée avec la propriété physique et/ou chimique du liquide de rinçage mesurée en mode de recirculation.

9. Procédé selon la revendication 8 **caractérisé en ce que** la propriété physique et/ou chimique du liquide de rinçage amené au système du liquide est mesurée en mode de rinçage.

10. Procédé selon la revendication 8 ou 9 **caractérisé en ce que** le système de liquide est commuté en mode de rinçage pour le rinçage libre avec du liquide de rinçage et la propriété physique et/ou chimique du liquide de rinçage est mesurée, commuté dans un premier mode de recirculation pour la recirculation de désinfectant et dans le mode de rinçage pour le rinçage forcé avec du liquide de rinçage, et commuté dans un deuxième mode de recirculation pour la recirculation du liquide de rinçage, et la propriété physique et/ou chimique du liquide de rinçage est mesurée.

11. Procédé selon l'une des revendications 8 à 10 **caractérisé en ce que** l'impureté à détecter dans le système de liquide est le désinfectant.

12. Procédé selon l'une des revendications 8 à 11 **caractérisé en ce que** la propriété physique et/ou chimique est la conductibilité du liquide de rinçage.

13. Procédé selon l'une des revendications 8 à 12 **caractérisé en ce que** le liquide de rinçage est l'eau.
